# EUROPEAN PATENT APPLICATION

(11) **EP 4 659 654 A1**
(43) Date of publication of application: **10.12.2025**
(21) Application number: 24179620.0
(22) Date of filing: 03.06.2024
(51) Int. Cl.: A61B 5/00

(54) **A PREGNANCY MONITORING SYSTEM AND METHOD**

(71) Applicant: Koninklijke Philips N.V., 5656 AG Eindhoven (NL)
(72) Inventor: BRAHMACHARI, Aveek Shankar, Eindhoven (NL); JOCHEMS, Anne, Eindhoven (NL)
(74) Representative: Philips Intellectual Property & Standards

(57) **Abstract**

A pregnancy monitoring system comprises an abdomen belt comprising at least one sensor for positioning against the abdomen of a pregnant woman. The signals captured by the at least one sensor are processed by a processor, which determines a proximity of a device worn on the hand or wrist of the pregnant woman relative to the at least one sensor. A signal is then provided to indicate a possible touch event when a distance between the device and the at least one sensor is below a threshold.

## Description

### FIELD OF THE INVENTION

The invention relates to a system and method for monitoring the progress of pregnancy.

### BACKGROUND OF THE INVENTION

The journey of a baby in an expectant mother's womb is the most wonderful thing nature has bestowed and thus, it is important to make sure that this journey is carried out safely leading to a healthy mother and baby.

Typically, most expectant mothers pay visits to doctors on a regular basis. However, increasingly mothers want to know about the well-being of their baby on a day-to-day basis. The tests that can be done in a laboratory cannot be done at home.

One proposed solution is to wear a belt having an array of sensors that monitors the activity of the baby. However, if the sensors are touched from outside, the measurements will get disturbed. Thus, to use IMUs for the purpose of fetal activity detection, a mechanism is needed that can be used for detecting any touch on the sensors from outside.

### SUMMARY OF THE INVENTION

The invention is defined by the claims.

According to examples in accordance with an aspect of the invention, there is a pregnancy monitoring system, comprising:
an abdomen belt comprising at least one sensor for positioning against the abdomen of a pregnant woman; and
a processor configured to process the signals captured by the sensors, wherein the processor is configured to:
   determine a distance between a device worn on the hand, wrist or arm of the pregnant woman and the at least one sensor; and
   provide a signal to indicate a possible touch event when a distance between the device and the at least one sensor is below a threshold.

This monitoring system prevents touch events creating false monitoring information by detecting those touch events, so that the associated sensor signals can be disregarded. The possible touch event is based on determining a distance between a hand of the expectant mother and one or more sensors of the belt. Thus, when the expectant mother cradles or otherwise holds her belly, the information provided by the sensor or sensors (or by other sensors) can be tagged as not providing information relating to the movement of the fetus. The system may further comprise the device, so that the abdominal belt and the device are part of the overall system.

A proximity measure may be obtained by monitoring the positions of the device and the positions of the at least one sensor, but an alternative is to use near field communication between the device and the sensors in order to determine more directly a distance between them.

The device for example may comprise a ring, watch, bracelet or glove.

The watch may of course be an existing smart watch of the expectant mother (with integrated IMU), and the processing communicates with the smart watch to obtain the IMU information.

The at least one sensor may comprise an array of inertial measurement units, IMUs, and the processor is configured to process the signals captured by the IMUs, to determine a proximity by:
deriving position information from an IMU signal of the device worn on the hand or wrist of the pregnant woman; and
determining a position of the device relative to the IMUs of the array.

In this way the sensors of the belt and the device track their positions over time, so that it can be determined when they come into close proximity.

The processor may be further configured to detect a possible touch event by determining that the direction of acceleration detected by an IMU of the array of IMUs is towards the fetus.

This provides an additional way to detect a touch event, based on the assumption that an inward pushing against the abdomen is likely to be caused by an external force rather than by the fetus.

The processor is for example further configured to perform a position re-calibration in response a signal received from the device, with the device at a predetermined position relative to one of the IMUs of the array. This cancels any signal drift due to the integration of acceleration signals to obtain spatial movement information.

The abdomen belt may further comprise a touch sensor. This provides an additional way to detect a touch event, based touching of the abdomen, for example when the expectant mother cradles or otherwise holds her belly. The touch sensor may comprise an array of capacitive touch sensors along the top front edge of the belt. There may of course be touch sensor at other locations where an expectant mother typically cradles her abdomen.

The processor is for example configured to generate pregnancy monitoring information which disregards the signals captured by the sensors of the array when a possible touch event is detected. The monitoring information for example relates to the size, orientation or activity of the fetus.

The invention also provides a pregnancy monitoring method, comprising:
determining a distance between a device worn on the hand or wrist of the pregnant woman and at least one sensor of an abdomen belt; and
providing a signal to indicate a possible touch event when a distance between the device and the at least one sensor is below a threshold.

In one example, determining a distance comprises:
receiving position information from an array of inertial measurement units, IMUs, of an abdomen belt positioned against the abdomen of a pregnant woman;
deriving position information from an IMU of the device worn on the hand or wrist of the pregnant woman; and
determining a position of the device relative to the IMUs of the array.

The method may comprise detecting a possible touch event by determining that the direction of acceleration detected by an IMU of the array of IMUs is towards the fetus.

The method may further comprise receiving a touch sensor input to determine a possible touch event.

The method may comprise performing a position re-calibration in response a signal received from the device, with the device at a predetermined position relative to one of the sensors of the array.

The method may comprise generating pregnancy monitoring information which disregards the signals captured by the sensors of the array when a possible touch event is detected.

The invention also provides a method of calibrating a pregnancy monitoring system, comprising:
during a calibration procedure assuming a known positional relationship between an abdomen belt of the pregnancy monitoring system and a device worn on the hand, wrist or arm, thereby to enable subsequent tracking of the position of the device relative to the abdomen belt.

The invention also provides a pregnancy monitoring method, comprising:
calibrating a pregnancy monitoring system using the method above; and
after the calibration, determining a distance between the device and the abdomen belt thereby to detect a possible touch event.

The invention also provides a computer program comprising computer program code which is adapted, when said program is run on a computer, to implement the method defined above. The computer program may be comprised in a computer program product.

The invention also provides a processor which is programmed with the computer program defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an abdomen belt;
Fig. 2 shows the belt worn by an expectant mother;
Fig. 3 shows the overall pregnancy monitoring system;
Fig. 4 shows a fetus modelled as an ellipsoid;
Fig. 5 shows how the ellipsoid changes in size and orientation over time; and
Fig. 6 shows the method performed by the processor to provide the monitoring function.
Fig. 7 shows the expectant mother's abdomen and shows the expectant mother wearing a device in the form of a watch;
Fig. 8 shows the expectant mother's abdomen and shows the expectant mother wearing a device in the form of a ring; and
Fig. 9 shows a pregnancy monitoring method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a pregnancy monitoring system that comprises an abdomen belt comprising at least one sensor for positioning against the abdomen of a pregnant woman. The signals captured by the at least one sensor are processed by a processor, which determines a proximity of a device worn on the hand or wrist of the pregnant woman relative to the at least one sensor. A signal is then provided to indicate a possible touch event when a distance between the device and the at least one sensor is below a threshold.

Typically, there are multiple sensors on the abdomen belt, and they are typically for monitoring movement of the fetus. In a first example described below, those sensors are additionally used to determine the proximity to a hand or wrist device worn by the expectant mother. However, in another example, the sensors of the belt may be proximity sensors (e.g. near field communication devices) specifically provided for determining the proximity to the hand or wrist worn device. In such a case, the abdomen belt will have other sensors for monitoring the fetus movement.

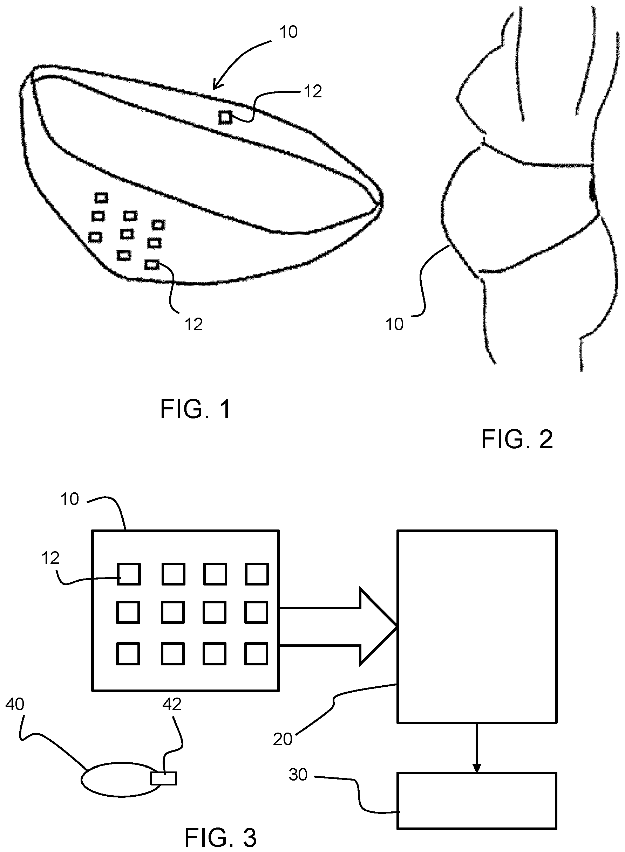

Fig. 1 shows an abdomen belt 10 of the first example comprising an array of inertial measurement units (IMUs) 12 for positioning against the abdomen of a pregnant woman. In the example shown, there is an array of IMUs for positioning over the belly and one at the back. The IMU at the back is for example a reference IMU, and the positions of all of the front IMUs are determined relative to the one at the back. However, any IMU may be used as a reference. Furthermore, at the limit there may be a single sensor on the abdomen belt.

The IMUs each comprise at least a 3-axis accelerometer so that their position may be tracked over time (by integration). Preferably, they also comprise a 3-axis gyroscope, so that position and orientation may be tracked over time. The IMUs also preferably include a magnetometer.

Fig. 2 shows the belt 10 worn by an expectant mother. The array of IMUs sits against the belly of the mother and thus the belt adopts the shape of the belly. It is flexible and stretchable to maintain contact with the belly while being comfortable to wear.

Fig. 3 shows the overall pregnancy monitoring system, comprising the belt 10, a processor 20 and an output interface 30. The output interface provides advice to the expectant mother. It may for example be a portable wireless device of the expectant mother (mobile phone or tablet) with which the processor 20 communicates over a wireless interface.

Fig. 3 also shows an external device 40 which also has an IMU 42. The external device 40 communicates with the processor 20 over a wireless communication channel. The external device may be the same device as the output interface 30 or it may be different. The external device is positioned on the hand or wrist of the expectant mother. It may comprise a ring, watch, bracelet or glove.

The processor 20 is configured to process the signals captured by the IMUs 12 as well as the signals captured by the IMU 42 of the device 40.

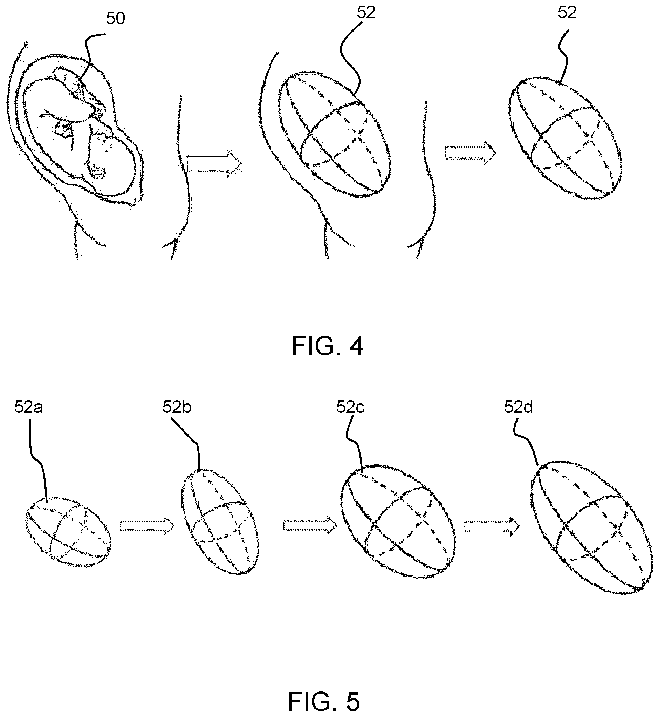

Fig. 4 shows a fetus 40. The fetus is modelled as an ellipsoid 50, and the size and preferably also orientation of that ellipsoid is monitored over time, by monitoring the shape of the abdomen of the expectant mother using the belt 10, which reports the positions of the IMUs relative to each other.

Fig. 5 shows how the ellipsoid changes in size and orientation over time (between size/orientations 50a, 50b, 50c and 50d).

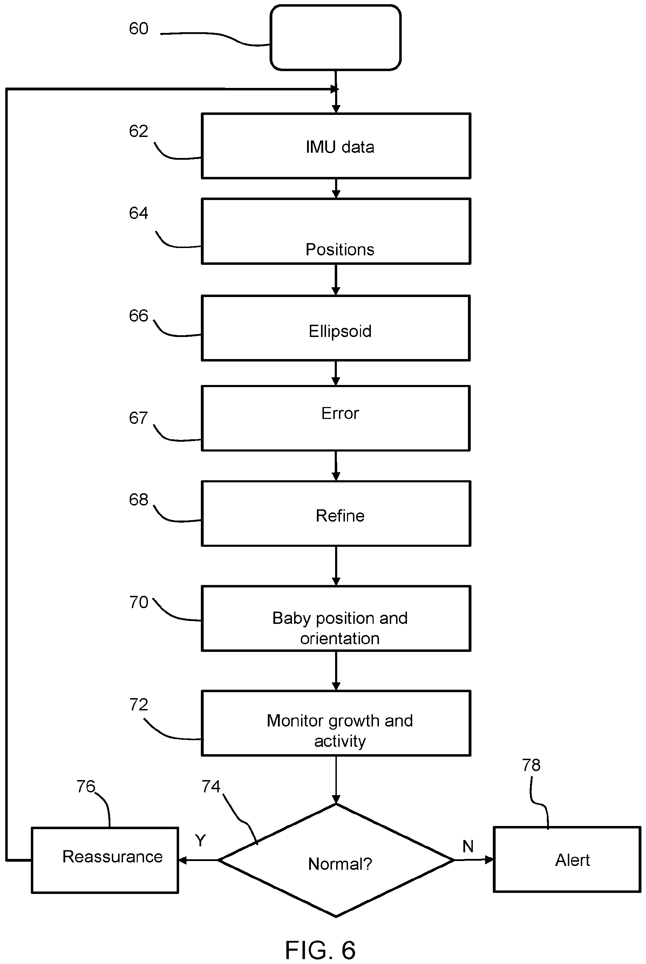

Fig. 6 shows the method performed by the processor to provide the monitoring function.

The process starts in step 60.

In step 62, streaming data from the IMUs 12 is captured by the processor 20.

In step 64, the positions of the IMUs are obtained relative to each other, for example relative to a reference IMU. For this purpose, a calibration process may have been carried out, by which the belt is applied to a template so that the relative positions of the IMUs are known. Movement of the IMUs can then be tracked by known methods, by cancelling the gravity vectors and integrating the acceleration vectors. By re-calibrating each time the belt is to be worn, signal drift can be reduced.

The IMU sensors report orientation information. The readings of the multiple sensors can be stabilized using sensor fusion by means of a Kalman filter. Before Kalman filtering, low pass filtering is performed to remove noise. Such sensor fusion approaches are for example well known for flying swarms of drones for air show purposes to avoid the need for frequent recalibration. The present application enables the use of additional geometric constraints such as the size of the belly or fetus, as these cannot change significantly over a single day, hence restricting the drift error.

In step 66, an ellipsoid shape is fitted to the determined positions. This is based on the assumption that the general area occupied by the fetus is an ellipsoid, and that the surface of the expectant mother's abdomen can be modelled as part of an ellipsoid. There is of course a scaling between the mother's abdomen and the fetus so that the fitted ellipse is not the same size as the fetus. However, tracking changes in the the size of the modelled ellipse can be used to track the growth of the fetus without any need for the scaling factor. Furthermore, a scaling factor can, if desired, be assumed between the abdomen ellipsoid size and the fetus size, and this scaling factor may take account of the expectant mother's physiology, such as the amount of subcutaneous fatty tissue, which may be indicated by a BMI measurement. A direct linear mapping of the fetus shape and size to the abdomen shape and size may be assumed, only differing in scale.

The baby's development can be tracked over a period. Because motion sensors are used for the position determination, the movement of the baby (that is detectable at the abdomen surface) can also be tracked.

In a most basic implementation, the size of the fitted ellipsoid shape can then be tracked over time (in step 72). However, some additional optional processing steps will also be described.

In step 67, an error between the ellipsoid shape and the determined IMU positions is determined, i.e., a fitting error. The fitting error can be aggregated for all IMUs. This gives an indication of how far the abdomen shape deviates from an ellipsoid. This information can have various uses. The position of the legs or head may for example be deduced from the modelling error of the ellipsoid. For example, a fetus with legs folded is shaped like a pear, with the head at the narrower end. Hence, the ellipsoid modelling has errors that may be used to indicate the positions of the head and/or legs.

In optional step 68, corrections may be made for errors in the IMU position information caused by signal drift. The orientations of the IMUs (using gyroscope sensors of the IMUs) can also help to find the center of the ellipse (for example where the normal axes of the IMUs meet or are closest to meeting). This center of the ellipse can help in refining the positions and orientations of the IMUs.

Thus, the IMU measurements can be refined using geometric constraints. as mentioned above, to reduce a fitting error. This refinement of the IMU data, by which sensor measurements are adjusted to provide correction of drift, can avoid the need for frequent recalibration of the belt.

One further approach is to use the Kalman filtered orientation to produce nearby points (arranged in a small square or circle around the center of the IMU). These points, together with the centers, give a better estimate of the ellipse. There may also be more points around a reference IMU, such as the IMU at the back of the belt.

In step 70, the orientation of the fetus is determined as well as the baby position (e.g., head up or down). The orientation as well as the size of the fitted ellipsoid shape can be monitored over time as shown in Fig. 5.

In addition, changes in the ellipsoid shape can be monitored over time using the fitting error obtained in step 67. A change in shape over short time periods may be used to detect movement of the fetus. A large error and a sudden change in shape would suggest fetal activity.

The orientation of the baby may include the fetus orientation (obtained from the major and minor axes of the fitted ellipsoid) but also the orientation of the baby within the fetus area (i.e. head up or down). For example, regions of greatest (local) movement may correspond to the legs and regions of least movement may correspond to the head. Local movements at the IMUs may also be used to derive a measure of the overall baby movement i.e., activity. Thus, local movements, rather than overall shape changes or baby orientation changes, may also be used to detect fetal movements.

In step 72 the movement and orientation information over time as well as the size information is processed to provide a measure of growth and activity level.

In step 74 it is determined if the monitored growth and activity is normal. If not, an alert is provided in step 78 (via the user interface 30). If everything is normal, reassurance is provided in step 76 (again via the user interface 30) and the monitoring continues.

Some of the IMU data from the abdominal belt may be corrupted by external touch events. This invention relates to the detection of those touch events. The IMU data corresponding to those touch events can then be disregarded so that they do not adversely affect the pregnancy monitoring results.

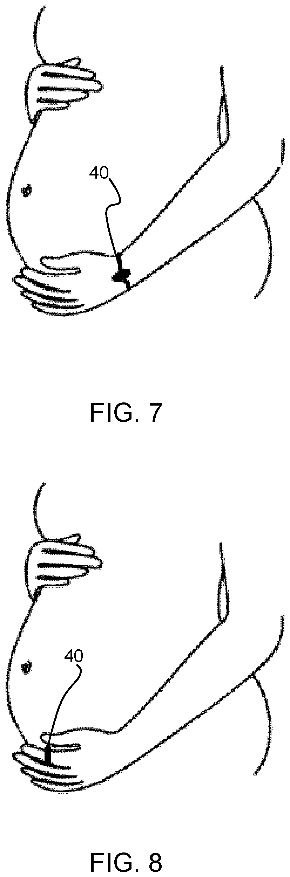

Fig. 7 shows the expectant mother's abdomen and shows the expectant mother wearing a device in the form of a watch.

Fig. 8 shows the expectant mother's abdomen and shows the expectant mother wearing a device in the form of a ring.

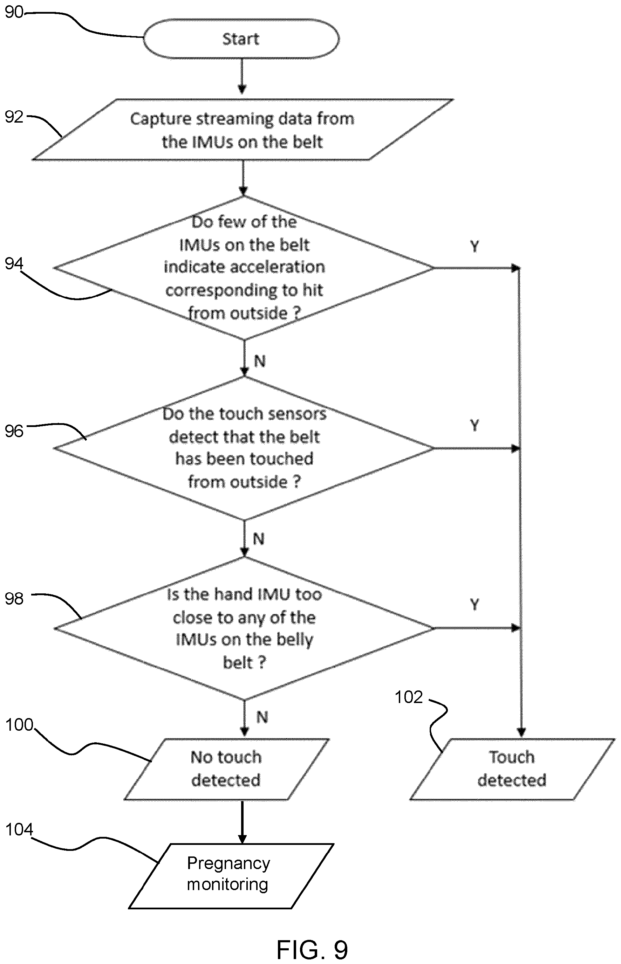

Fig. 9 shows a pregnancy monitoring method.

The method starts in step 90.

In step 92, position information is received from the array of IMUs of the abdomen belt positioned against the abdomen of a pregnant woman.

The three approaches discussed above for touch detection are shown.

In step 94 it is determined if the acceleration direction for any of the belt IMUs is towards the fetus, corresponding to a hit from outside.

In step 96, a touch event is detected using touch sensors.

In step 98, position information of the IMU 42 of the device 40 worn on the hand or wrist of the expectant mother is obtained. The position of the device 40 is derived relative to the IMUs of the array. It can then be determined if the IMU of the device 40 is too close to any of the IMUs of the abdominal belt, such as to indicate a possible touch event. If a distance between the device 40 and any IMU of the array is below a threshold then a possible touch event is detected.

When any of the three touch event sensing approaches senses a possible touch event, a touch detection signal is generated in step 102. A signal is provided to indicate the possible touch event. The signal for example tags the IMU data at that time so that it is not used for the pregnancy monitoring as explained above.

When there is no touch detected (in step 100), pregnancy monitoring information is generated in step 104. Because the IMU data associated with the touch events is discarded, this pregnancy monitoring disregards the signals captured by the IMUs of the array when a possible touch event is detected. The pregnancy monitoring may be as explained above, but any method of interpreting the IMU data may be employed.

Advice may be provided to the expectant mother that the IMU signals are not being recorded because of the external touching. This advice may for example be provided after a certain time delay.

To enable accurate relative positions between the external device (ring, watch etc.) and the abdomen belt, a recalibration can be performed periodically. For example, the user can place the external device against a selected one of the IMUs and send a signal to perform calibration (by pressing a button on the external device). The system then knows the relative position between the external device and the selected IMU so that offsets can be prevented.

In this way, the pregnancy monitoring system, comprising an abdomen belt and a device worn on the hand, wrist or arm, is calibrated such that a position relationship between the abdomen belt and the device worn on the hand, wrist or arm is set to a known relationship. In this way, it can be detected when the device is positioned against the abdomen belt.

In addition to movement and shape of the fetus, the IMUs may be used to monitor the expectant mother's breathing rate, and movement frequencies at the breathing rate may be filtered out of the IMU signals.

The IMU data may be processed with normalization and non-maximal suppression. Regions of fetal movement may be localized and patterns in position and magnitude may be analyzed to narrow down to possible fetal movements.

The activity may be presented as a movement percentage, and this may be provided as a report when triggered by the expectant mother.

When the device comprises a smart watch, additional functionality may be enabled. For example, the expectant mother's heart rate may be monitored, or other vital signs may be measured.

The example above uses an abdomen belt with IMU sensors for monitoring fetal movement as well as for monitoring proximity to the hand or wrist of the expectant mother. The belt may instead have dedicated proximity sensors (in addition to other fetus monitoring sensors) and these dedicated proximity sensors are used to monitor how close the hand or wrist worn device comes to the sensors. Near field communication may be used to determine a distance between communicating devices based an a signal strength measurement. Thus, the belt may the combine NFC sensors for proximity sensing and IMU sensors for fetal movement monitoring. When proximity is detected using the NFC sensors, the movement information collected by the IMUs can be disregarded.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A pregnancy monitoring system, comprising:
an abdomen belt (10) comprising at least one sensor for positioning against the abdomen of a pregnant woman; and
a processor (20) configured to process the signals captured by the at least one sensor, wherein the processor is configured to:
(98) determine a distance between a device worn on the hand, wrist or arm of the pregnant woman and the at least one sensor; and
(102) provide a signal to indicate a possible touch event when a distance between the device and the at least one sensor is below a threshold.

2. The system of claim 1, wherein the at least one sensor comprises an array of inertial measurement units (12), IMUs, and wherein the processor (20) is configured to process the signals captured by the IMUs, to determine a proximity by:
deriving position information from an IMU signal of the device worn on the hand or wrist of the pregnant woman; and
(98) determining a position of the device relative to the IMUs of the array.

3. The system of claim 2, wherein the processor (20) is further configured to detect a possible touch event by determining that the direction of acceleration detected by an IMU of the array of IMUs is towards the fetus.

4. The system of any one of claims 2 to 3, wherein the processor (20) is further configured to perform a position re-calibration in response to a signal received from the device, with the device at a predetermined position relative to one of the IMUs of the array.

5. The system of any one of claims 1 to 4, further comprising the device (40).

6. The system of any one of claims 1 to 5, wherein the abdomen belt further comprises a touch sensor.

7. The system of claim 6, wherein the touch sensor comprises an array of capacitive touch sensors along a top front edge of the belt.

8. The system of any one of claims 1 to 7, wherein the processor is configured to generate pregnancy monitoring information which disregards the signals captured by the at least one sensor when a possible touch event is detected.

9. A pregnancy monitoring method, comprising:
determining a distance between a device worn on the hand, wrist or arm of the pregnant woman and at least one sensor of an abdomen belt; and
providing a signal to indicate a possible touch event when a distance between the device and the at least one sensor is below a threshold.

10. The method of claim 9, wherein determining a distance comprises:
receiving position information from an array of inertial measurement units, IMUs, of the abdomen belt positioned against the abdomen of a pregnant woman;
deriving position information from an IMU of the device worn on the hand or wrist of the pregnant woman; and
determining a position of the device relative to the IMUs of the array.

11. The method of claim 10, comprising detecting a possible touch event by determining that the direction of acceleration detected by an IMU of the array of IMUs is towards the fetus.

12. A method of calibrating a pregnancy monitoring system, comprising:
during a calibration procedure, assuming a known positional relationship between a sensor for an abdomen belt of the pregnancy monitoring system and a device worn on the hand, wrist or arm, thereby to enable subsequent tracking of the position of the device relative to the abdomen belt.

13. A computer program comprising computer program code which is adapted, when said program is run on a computer, to implement the method of any one of claims 9 to 12.

14. A processor which is programmed by the computer program of claim 13.

15. Use of a device worn on a hand, wrist or arm of a pregnant woman in a pregnancy monitoring system of any one of claims 1 to 8.
